# EUROPÄISCHE PATENTSCHRIFT

(11) **EP 0 906 379 B1**
(45) Veröffentlichungstag und Bekanntmachung des Hinweises auf die Patenterteilung: **23.04.2003**
(21) Anmeldenummer: 96921886.6
(22) Anmeldetag: 04.07.1996
(51) Int. Cl.: C11B 3/00, C12N 9/16

(54) **ENZYMATISCHES VERFAHREN ZUR ENTSCHLEIMUNG VON PFLANZLICHEN ÖLEN MIT ASPERGILLUS-PHOSPHOLIPASE**
VEGETABLE OIL ENZYMATIC DEGUMMING PROCESS BY MEANS OF ASPERGILLUS PHOSPHOLIPASE
PROCEDE DE DEMUCILAGINATION PAR VOIE ENZYMATIQUE D'HUILES VEGETALES AU MOYEN DE PHOSPHOLIPASE D'ASPERGILLUS

(30) Priorität: 26.07.1995 DE 19527274
(43) Veröffentlichungstag der Anmeldung: 07.04.1999
(73) Patentinhaber: AB Enzymes GmbH, 64293 Darmstadt (DE); MG Technologies AG, 60325 Frankfurt am Main (DE)
(72) Erfinder: LÖFFLER, Fridolin, D-64625 Bensheim (DE); PLAINER, Hermann, D-64354 Reinheim (DE); SPRÖSSLER, Bruno, D-64380 Ro dorf (DE); OTTOFRICKENSTEIN, Hans, D-64673 Zwingenberg (DE)
(74) Vertreter: Banzer, Hans-Jörg, Dipl.-Ing.
(86) Internationale Anmeldenummer: DE9601190
(87) Internationale Veröffentlichungsnummer: WO97005219

(56) Entgegenhaltungen:
- EP-A- 0 219 269
- EP-A- 0 513 709
- EP-A- 0 575 133
- EP-A- 0 622 446
- US-A- 5 288 619
- US-A- 5 314 706
- PROCESS BIOCHEMISTRY, Bd. 30, Nr. 5, 1995, GB, Seiten 393-401, XP000603907 A. MUSTRANTA ET AL.: "Comparison of lipases and phospholipases in the hydrolysis of phospholipids"
- DATABASE WPI Week 9304 Derwent Publications Ltd., London, GB; AN 93-030369 XP002014555 & JP,A,04 356 192 (KANEKA CORP) , 9.Dezember 1992
- DATABASE WPI Week 9304 Derwent Publications Ltd., London, GB; AN 93-030368 XP002014556 & JP,A,04 356 191 (KANEKA CORP) , 9.Dezember 1992
- DATABASE WPI Week 9507 Derwent Publications Ltd., London, GB; AN 95-047898 XP002014557 & JP,A,06 327 475 (NISSHIN OIL MILLS LTD) , 29.November 1994
- DATABASE WPI Week 8705 Derwent Publications Ltd., London, GB; AN 87-033297 XP002014558 & JP,A,61 289 884 (ASAHI DENKA KOGYO) , 19.Dezember 1986

## Beschreibung

### Gebiet der Erfindung

Die Erfindung betrifft den Verfahrensschritt der Entschleimung bei der Herstellung von Speiseölen, wobei pflanzliche Öle, denen die hydratisierbaren Phosphatide vorzugsweise durch eine vorgeschaltete wäßrige Entschleimung weitgehend entzogen worden waren, durch eine Enzymbehandlung von nicht hydratisierbaren Phosphatiden soweit befreit werden, daß sie einer physikalischen Raffination unterzogen werden können. Das Verfahren ist schonend, kostensparend und umweltfreundlich.

### Stand der Technik

Die anerkannten Raffinationsverfahren zur Herstellung von Speiseölen höchster Qualität umfassen üblicherweise die Verfahrensschritte der Entschleimung, der Entsäuerung, sowie der Bleichung und der Desodorierung. Große Anstrengungen wurden in letzter Zeit unternommen, um insbesondere das Entschleimungsverfahren effizienter und kostengünstiger zu gestalten. Das angestrebte Ziel dabei ist, das Öl soweit zu entschleimen, daß es anschließend destillativ entsäuert werden kann. Letzteres destillatives Entsäuerungsverfahren hat gegenüber dem herkömmlichen Verfahren der Entsäuerung durch Neutralisation den großen Vorteil, daß kein Abfall entsteht. Voraussetzung für seine Durchführung ist aber eine sehr niedriger Gehalt an Phosphatiden, z.B. ein Phosphorgehalt von weniger als 15ppm im Öl, bevorzugt von weniger als 10 ppm. Ideal sind Phosphorgehalte von <5 ppm.

Die Schleimstoffe der Pflanzenöle bestehen hauptsächlich aus Gemischen von Phosphatiden, deren Menge und Zusammensetzung von der Ölsaat und der Art der Ölgewinnung abhängt. Der weitaus größte Teil der Phosphatide läßt sich durch Hydratation aus ihren micellaren Lösungen in den Rohölen abtrennen und zur Lecithingewinnung verwenden. Man spricht dabei von Naßentschleimung. Ein kleiner Teil der Phosphatide ist nicht hydratisiertbar und verbleibt im Öl. Die chemische Natur dieser "nicht hydratisierbaren Phosphatide (NHP) ist nicht restlos aufgeklärt. Untersuchungen haben ergeben, daß sie zu über 50% aus Calcium- und Magnesiumsalzen von Phosphatidsäuren bestehen (Siehe Hermann Pardun, Die Pflanzenlecithine, Verlag für chem. Industrie H. Ziolkowsky KG, Augsburg, 1988, Seite 181).

Das Ziel gängiger technischer Entschleimungsverfahren ist es, die nicht hydratisierbaren Phosphatide so weit wie möglich aus dem Öl zu entfernen. Zu den heute üblichen Verfahren gehören das "Superdegumming Verfahren" und das "Unidegumming" Verfahren von der Firma Unilever, das "Total Degumming ("TOP") Verfahren" der Firma Vandemoortele, das "Alcon-Verfahren" der Firma Lurgi und das "UF-Verfahren" der Firma Krupp Maschinentechnik GmbH.
Vielfach ist die klassische Wasserentschleimung zur Entfernung der hydratisierbaren Phosphatide in diesen Verfahren integriert oder ihnen vorgeschaltet.

Typisch für alle diese Enschleimungsverfahren ist, daß ausschließlich rein mechanische oder physikalisch-chemische Methoden angewandt werden, die nicht immer für alle Ölqualitäten optimal geeignet sind. Der apparative Aufwand und der Energieaufwand sind für diese Verfahren hoch, zudem ist nicht garantiert, daß die für eine nachfolgende destillative Entsäuerung notwendigen niedrigen Phosphorgehalte erzielt werden.

Bei einem Teil dieser Entschleimungsverfahren wird als wirksames Prinzip eine Säurebehandlung angewandt. Es ist bekannt, daß stark saure Agentien zur Nachentschleimung von mit Wasser vorentschleimten Ölen geeignet sind (vgl.Pardun, loc. cit , Seiten 185-189 oder US-A 4 698 185). Bevorzugt wird dabei Citronensäure eingesetzt.

In der europäischen Patentanmeldung 0 513 709 wird erstmals ein wirksames enzymatisches Verfahren zur Entschleimung vorgestellt. Dabei wird ein mit Wasser vorentschleimtes Speiseöl mit einer wäßrigen Lösung einer Phospholipase (A₂, A₁, B) emulgiert und von dieser wäßrigen Phase abgetrennt. Das Öl enthält nach diesem Prozeß weniger als 5 ppm Phosphor und ist für die nachfolgende destillative Entsäuerung geeignet. Wichtige Prozeßparameter sind eine Emulgierung der enzymhaltigen Wasserphase zu Tröpfchen < 10 Mikrometer, der Zusatz von Citrat zur wäßrigen Lösung, eine Temperatur von 50 bis 70 C, und ein pH-Wert bevorzugt von 4 bis 6. Diese pH-Einstellung im Sauren ist überraschend, denn die pH-Optima aller bekannten Phospholipasen liegen bei pH 8. Das enzymatische Entschleimungsverfahren wurde als "EnzyMax-Verfahren" von der Firma Lurgi in der Speiseölindustrie eingeführt.

In der DE-A 43 39 556 wird als weitere Variante dieses Verfahrens die Wiederverwendung des Enzyms beschrieben, indem man es aus einer gebrauchten, schlammhaltigen wäßrigen Phase durch Zusatz von Tensiden oder Lösungsvermittler ablöst und als weitgehend schlammfreie Lösung, die mindestens 10% des eingesetzten Enzyms enthält, wiedergewinnt.

Im "EnzyMax-Verfahren" kann die vorteilhafte Wirkung der Citronensäure für eine weitgehende Entschleimung benützt werden, und zwar durch eine der Enzymbehandlung vor- oder nachgeschaltete Citronensäurebehandlung. Der gleichzeitige Einsatz von Citronensäure und Enzym ist nicht möglich.

Aus der JP-A 2-153997 ist es bekannt, rohes oder vorentschleimtes Öl mit einem Enzym, das Phosphoplipase-A-Aktivität aufweist, zu behandeln. Dieser Stand der Technik lehrt. daß durch den Einsatz von Phospholipase A die Phosphatide so verändert werden, daß sie durch Adsorbentien wie Aktivton oder Bleicherde leicht entfernt werden können. So wird in Beispiel 1 und 2 von 3 Durchführungsbeispielen die Enzymbehandlung mit einer Bleicherdebehandlung kombiniert. Im 3. Beispiel wird auf den Einsatz von Bleicherde verzichtet .Statt dessen werden hier besonders große Mengen Enzym (2000-20000 Einheiten) in großen Mengen Wasser (100-1000 Gew.-% bez. auf das Öl) eingesetzt. Dabei stellt sich eine Ölin-Wasser-Emulsion ein. Es wird keine Lehre zur Verteilung des Öls in der enzymhaltigen Wasserphase, zur Einstellung des pH-Wertes, zur Mitverwendung von Citrat, sowie zur Wiederverwendung des Enzyms gegeben.

In der JP-A 2-49593 wird eine ähnliche Enzymbehandlung von Ölen beschrieben, die indessen nicht auf die Entschleimung des Öls, sondern auf die Gewinnung von Lysolecithin abzielt. Dabei ist die Einstellung besonderer pH-Werte überflüssig.

Auch beim Verfahren gemäß EP-A 0 328 789 geht es um die Umwandlung des Lecithins des Sojaöls zu Lysolecithin durch Phospholipase A zur Herstellung von mayonnaise-artigen Produkten.

Die EP-A 0 622 446 beschreibt ein enzymatisches Verfahren zur Entschleimung von Öl und Fett, das mehrere Verfahrensschritte beinhaltet. Nach der Behandlung mit Phospholipase wird die Enzymlösung abzentrifugiert, das verbleibende Öl mit Wasser von pH 3-6 gewaschen und schließlich mit Bleicherde behandelt. Kennzeichnend ist dabei, daß sowohl bei der Enzymbehandlung, als auch beim Waschschritt große Mengen Wasser, nämlich 30-200 Gewichts-% bezogen auf das eingesetzte Öl eingesetzt werden. Auch hier entstehen Öl-in-Wasser-Emulsionen. Dies bedeutet erhöhten apparativen Aufwand, denn es müssen große Volumina an Flüssigkeit bewegt werden, sowie höhere Energie- und Entsorgungskosten. Für die pH-Einstellung der wäßrigen Enzymlösung wird keine Lehre gegeben.

Die Bereitstellung der erforderlichen Menge an Enzym für die Betreibung eines großtechnischen enzymatischen Verfahrens ist ein spezifisches Problem im Falle der Phospholipase. Hier ist die verfügbare Menge beschränkt. Phospholipase A₁ ist nicht im Handel erhältlich, Phospholipase B nur in Labormengen; Quellen sind Extrakte aus Rattenleber oder aus Streptomyces-Kulturen. Phospholipase A₂ kommt im Schlangen-, Skorpions- und Bienengift vor.
Alle diese Quellen sind nicht für die Erzeugung technisch relevanter Enzymmengen geeignet. Das derzeit einzige technische Herstellverfahren für Phospholipase A₂ ist die Extraktion aus Schweine-Pankreasdrüsen. Weltweit ist das Aufkommen an geeigneten Drüsen aber eng begrenzt und auf keinen Fall beliebig vermehrbar. Zudem ist Phospholipase bei dem Extraktionsverfahren nur ein untergeordnetes Nebenprodukt. Hauptprodukte sind Pankreasproteasen, insbesondere Trypsin, sowie auch Schweineinsulin. Es wird geschätzt, daß das derzeitige - und kaum vermehrbare - Handelsvolumen an Phospholipase A₂ für nicht mehr als zwei bis drei Ölmühlen ausreichen würde, selbst wenn man das Enzym, wie in EP-A 0 513 709 beschrieben, im Prozeß wiederverwendet.

Die EP 0 575 133 betrifft eine Phospholipase A₁, die aus Aspergillus erhältlich ist, sowie ein Verfahren zum Erhalt dieses Enzyms. Diese Druckschrift enthält jedoch keinen Hinweis auf ein Verfahren zur Verminderung der in pflanzlichen Ölen enthaltenen phosphorhaltigen Bestandteile.

Die US-PS 5 314 706 betrifft ein Verfahren zur Herstellung von stabilen Öl-in-Wasser-Emulsionen, wobei Lysophospholipide aus Eigelb als Emulgator verwendet werden. Diese werden mit Hilfe von Pancreaslipase A₂ aus Sojalecithin hergestellt. Dieses Verfahren ist kein Verfahren zur Entschleimung von pflanzlichen Ölen.

Die Druckschrift Process Biochemistry, Vol. 30, Nr. 5, Seiten 393-401, 1995 betrifft einen Vergleich verschiedener Lipasen und Phospholipasen aus Aspergillus niger, Penicillium cyclopium und Schweinepancreas. Beschrieben werden die katalytischen Aktivitäten der Enzyme sowie ihre physikalischen Eigenschaften. Diese Druckschrift enthält keinen Hinweis auf ein Verfahren zur Entschleimung bei der Herstellung von Speiseölen.

Es besteht somit Bedarf nach einer Quelle, die das Enzym in unbeschränkter Menge bereitstellt. Technische Enzyme werden nach dem Stand der Technik in beliebiger Menge aus Mikrorganismen, z.B. aus Pilzen oder Bakterien gewonnen. Für Phospholipasen A₁, A₂ und B ist derzeit kein Mikroorganismus bekannt, der die Produktion dieses Enzyms mit ausreichender Ausbeute lohnt. Isoliert wurde Phospholipase A₁ aus Rhizopus arrhizus, Escherichia coli und Bacillus megaterium, Phospholipase B aus Penicillium notatum und Streptomycesstämmen. Überraschenderweise findet sich in der Literatur kein Hinweis, daß Phospholipase A₁, A₂ oder B aus Aspergillus isoliert werden kann.

Dagegen sind Lysophospholipasen, die aus Aspergillus niger gewonnen werden, aus EP 0 219 269 bekannt. Lysophospholipasen - sie werden hier als Phospholipase L₁ bzw. L₂ bezeichnet - besitzen eine gegenüber den oben genannten Phospholipasen unterschiedliche Spezifität insofern, als sie ausschließlich Monoacylphosphatide zu spalten vermögen, wie z.B. Lysolecithin. Reine Lysophospholipasen finden auf einem ganz anderen Gebiet der Lebensmitteltechnologie Anwendung, nämlich zur Verbesserung der Ausbeute bei der Weizenstärkefiltration.

### Aufgabe

Aufgabe der vorliegenden Erfindung ist es, ein schonendes, umweltfreundliches und kostengünstiges Verfahren zur Verminderung des Gehaltes an phosphorhaltigen Bestandteilen in pflanzlichen Ölen bis zu einem Grad, der eine Weiterbehandlung des Öles durch destillative Entsäuerung ermöglicht, also bis zu Phosphorgehalten von weniger als 15 ppm, bevorzugt von weniger als 10 ppm, im besten Fall von weniger als 5 ppm bereitzustellen. Diese Forderungen können durch ein enzymatisches Verfahren erfüllt werden.

Es besteht dabei Bedarf nach einer mikrobiellen Quelle, die erlaubt, das Enzym Phospholipase in unbeschränkter Menge zu produzieren. Nach dem Stand der Technik sind ausschließlich Enzyme mit acylspaltender Spezifität dafür brauchbar, nämlich. Phospholipase A₁, A₂, und B. Dabei ist es von nicht zu unterschätzendem Vorteil, einen enzymproduzierenden Mikroorganismus zu verwenden, der in der Lebensmitteltechnologie seit langem eingeführt und daher unbedenklich ist. Beispiele hierfür sind verschiedene Hefestämme wie Kluyveromyces cerivisiae, Bacillusstämme wie B. subtilis oder Aspergillusstämme wie A. niger oder oryzae.

Eine weitere, bisher nicht gelöste Aufgabe ist es, im Entschleimungsprozeß die vorteilhafte Wirkung von zwei bekannten Prozessen, nämlich die der Säurebehandlung und der Enzymbehandlung, in einem Schritt, zu vereinigen.

Schließlich stellt sich die Aufgabe, mit möglichst geringen Einsatzmengen an Enzym und Säure auszukommen, um dadurch das Verfahren besonders wirtschaftlich zu gestalten zu können.

### Lösung

Die Aufgabe wird gelöst durch ein Verfahren zur Verminderung von in pflanzlichen Ölen enthaltenen phosphorhaltigen Bestandteilen durch deren enzymatischen Abbau mit acylspaltenden Phospholipasen, dadurch gekennzeichnet, daß ein aus Aspergillus stammendes Enzym eingesetzt wird, wobei das Enzym sowohl Phospholipase A₂-Aktivität (EC - Nr. 3.1.1.4) und/oder Phospholipase A₁-Aktivität (EC - Nr. 3.1.1.32), als auch Lysophospholipase-Aktivität (EC - Nr. 3.1.1.5) enthält und der pH-Wert der eingesetzten Enzymlösung auf < 3 eingestellt wird.

Die Herstellung von technischen Enzymen durch Kultivierung von Aspergillus-Stämmen ist ein wichtiger und hochentwickelter Bereich in der Biotechnologie. So werden Enzyme aus Aspergillus niger u. a. in der Stärkeindustrie (Amyloglucosidasen), in der Fruchtsaftindustrie (Pectinasen) und in der Backwarenindustrie (Xylanasen) in großem Maßstab eingesetzt. Enzyme aus Aspergillus, insbesondere aus A. niger sind in der Lebensmitteltechnologie schon lange eingeführt und als unbedenklich bekannt. Phospholipasen A₁, A₂, oder B aus dieser Quelle sind nicht bekannt. Die Verwendung der beanspruchten Phospholipasen dieser Herkunft im Entschleimungsverfahren ist ein wichtiges Kennzeichen der vorliegenden Erfindung. Vorausgegangen war die Suche nach Phospholipase enthaltenden Stämmen durch die an sich bekannte Screeningmethode der Stammverbesserung durch Mutation und Selektion auf erhöhte Phospholipase Aktivität.

Die Spezifität der aus dieser Quelle gewonnenen Phospholipase kann vielseitig und komplex sein. Anders als bei der aus dem Stand der Technik bekannten Phospholipase A₂ aus Pankreas, die definitionsgemäß nur die Acylgruppe am C₂-Atom eines Phospholipidmoleküls spaltet, enthalten erfindungsgemäß verwendete Phospholipasen aus Aspergillus verschiedene acylspaltende Spezifitäten gleichzeitig, nämlich neben einer A₁- und A₂-Spezifität auch eine Lysophospholipase-Aktivität.

Den genannten Enzymspezifitäten entsprechen die E.C.-Nummern 3.1.1.32, 3.1.1.4 und 3.1.1.5. Lysophospholipase (EC-Nr. 3.1.1.5) wird auch als Phospholipase B bezeichnet; es ist aber nach Darstellung in der Literatur (vgl. Pardun, loc.cit., Seite 140) unklar, ob zwischen Lysophospholipase und Phospholipase B unterschieden werden muß. Gemeinsam ist ihnen jedenfalls, daß sie Lysolecithin weiter zu spalten vermögen und zwar bis zum Glycerophosphorylcholin. Phospholipase B vermag zusätzlich auch Lecithin aunzugreifen. Da die erfindungsgemäße Phospholipase diese beiden Spezifitäten besitzt, nämlich sowohl für das Substrat Lecithin, als auch für das Substrat Lysolecithin, könnte man sie als Phospholipase B bezeichnen. Reine Lysophospholipasen aus Aspergillus, die nur Lysolecithin zu spalten vermögen, nicht aber Lecithin, sind im vorliegenden Entschleimungsprozeß, insbesondere unter den sauren Reaktionsbedingungen, nach den bisherigen Erkenntnissen der Anmelderin wirkungslos. Das gilt auch für die nicht acylspaltenden Phospholipasen C und D.
Somit ist die Spezifität für Lecithin, also Phospholipase A₁ und/oder A₂ Aktivität - es ist schwer, analytisch zwischen beiden zu unterscheiden - ein wesentliches Kennzeichen des erfindungsgemäßen Enzyms.
Das gleichzeitige Vorhandensein dieser verschiedenen Spezifitäten könnte ein Grund für die vorteilhafte Wirkung des erfindungsgemäßen Enzyms sein. Obwohl es in den meisten Fällen als Einzelenzym bezeichnet werden muß, besitzt es die Wirkung eines Enzymkomplexes.

Beim Einsatz des erfindungsgemäßen Enzyms ist sein Reinigungsgrad ohne große Bedeutung. Es können z.B die Fermentationsflüssigkeit selbst, das nach einer Ultrafiltration erhaltenen enzymreichere Retentat oder das daraus gefällte Enzymprotein eingesetzt werden.

Es ist durchaus im Sinne der Erfindung, anstelle des aus einem konventionellen Aspergillus-Produktionsstamm gewonnenen Enzyms ein aus einem gentechnisch veränderten Produktionsstamm gewonnenes Enzym zu verwenden. Die Gentechnik bietet inzwischen eine Vielzahl von Möglichkeiten, das für die Bildung der Phospholipase benötigte Gen aus Aspergillus zu klonieren und in einem geeigneten Wirtstamm in hoher Ausbeute zu exprimieren. Als Wirtsstämme kommen z.B. wieder Aspergillusstämme, aber auch andere Pilzstämme und sogar Bakterienstämme in Frage.

Die Einsatzmengen des Enzyms können entsprechend seinem Reinigungsgrad zwischen 0.0001 bis 1% bezogen auf das zu entschleimende Öl liegen.

Ein großer Vorteil und ein unerwarteter Effekt der erfindungsgemäß verwendeten Enzyme ist die für die Durchführung der Entschleimung notwendige Aktivität: Sie ist außerordentlich klein. Während im Stand der Technik bei Verwendung von Phospholipase A₂ aus Pankreas Aktivitäten von ca. 1000 Lecitase-Units (LU) pro 1 Liter Öl eingesetzt wurden (Siehe Beispiel 1 in der EP-A 0 513 709), genügen bei Verwendung der erfindungsgemäßen Enzyme aus Aspergillus unter den oben beschriebenen Bedingungen Aktivitäten von nur 5 bis 50 LU.pro Liter Öl. Bei entsprechend verlängerter Reaktionszeit sind sogar Einsatzmengen von unter 5 LU pro Liter Öl zielführend.
Die in gereinigten Aspergillus-Phospholipasen gefundene Lysophospholipase-Aktivität liegt sogar höher als die Phospholipase A₂-Aktivität, und zwar um das 1 bis 100fache. Dadurch ergeben sich Einsatzmengen von 5 bis 5000 Lysolecitase-Units (LLU) pro Liter Öl, bevorzugt 50 bis 1000 LLU. Diese Aktivitätsangaben gelten für batchweise Entschleimungsansätze. Bei Wiederverwendung des Enzyms liegen die notwendigen Enzymzusätze bezogen auf 1 Liter Öl bedeutend niedriger, z.B bei 1/5 bis 1/10 der obigen Werte. Diese geringen Enzymmengen erlauben es, daß auf eine Wiederverwendung des Enzyms auch verzichtet werden kann.

Es ist möglich, die erfindungsgemäße Aspergillus Phospholipase mit anderen acylspaltenden Phospholipasen gezielt abzumischen, z.B. mit weiteren Phospholipasen aus Aspergillus oder mit Phospholipase A₂ aus Pankreas. Im letzteren Fall muß allerdings ein pH-Wert eingestellt werden, der beiden pH-Optima nahekommt, also pH < 3.

Die Verwendung von Phospholipase aus Aspergillus ermöglicht überraschenderweise auch die Lösung weiterer gestellter Aufgaben. So ist es möglich, das Enzym in einer Citroriensäurelösung einzusetzen, die Wirkung des Enzyms mit dem der Citronensäure zu vereinigen. Die Anwendung eines Enzyms in relativ konzentrierten Citronensäurelösungen ist ungewöhnlich. In der Enzymologie sind kaum Enzyme bekannt, die bei so tiefen pH-Werten stabil sind und hier sogar ihr Wirkungsoptimum besitzen. Eines der wenigen Beispiele für ein derartiges Eigenschaftsspektrum ist das Pepsin des Verdauungstraktes.
Das Enzym wird in einer 1-20%igen Citronensäurelösung gelöst. Dabei stellt man pH-Werte von pH<3 ein. Verwendet man z.B. 5%ige Citronensäurelösungen, so ergibt sich ein pH-Wert von ca. 2.3.

Anstelle von Citronensäure können auch Milchsäure, Essigsäure, Ameisensäure, Phosphorsäure, sowie andere anorganische und organische Säuren verwendet werden. Bevorzugt sind aber sind Genußsäuren, insbesondere Citronensäure. Anzumerken ist, daß mit Säurelösungen allein, also ohne Einsatz des Enzyms, keine ausreichend niedrigen Phosphorgehalte erzielt werden, insbesondere bei vorentschleimten Ölen.

Das im erfindungsgemäßen Verfahren eingestellte pH-Optimum von 2-3 stimmt nicht mit dem nach üblichen analytischen Verfahren gefundenen pH-Optimum überein. Letzteres liegt bei pH=8, wobei Eigelb als Substrat bei 40°C in der Enzymlösung emulgiert wird und die Aktivität in Abhängigkeit vom pH-Wert bestimmt wird. Das überraschend niedrige pH-Verfahrensoptimum könnte man mit den besonderen Bedingungen der Phasengrenzfläche erklären, wo sich vielleicht ein höherer pH-Wert einstellt, als er in der Wasserphase ("bulk-Phase") gemessen wird.

Das Enzym wird in dieser sauren wäßrigen Lösung mit dem Öl innig vermischt. Dabei sollte man bestrebt sein, die Wasserphase im Vergleich zur Ölphase möglichst klein zu halten, um bei deren anschließendem Abscheiden möglichst kleine Volumina bewegen zu müssen. In der Regel kommt man mit Volumina < 10% bezogen auf die Ölphase aus, bevorzugt mit Volumina <5%. In jedem Fall entsteht eine Wasser-in-ÖI-Emulsion.

Die zu behandelnde Ölphase kann Sojaöl sein, Sonnenblumenöl oder Rapsöl. Ersteres ist das wichtigste Ölprodukt. Andere pflanzliche Öle, wie Leinöl, Cocosöl oder Palmöl, sowie tierische Öle, die störende Phosphatide enthalten, können ebenfalls nach dem erfindungsgemäßen Verfahren behandelt werden.

Da die Phospholipase Lecithin angreifen würde, ist es nicht sinnvoll, hoch lecithinhaltige Öle, wie rohes Sojaöl, in dem erfindungsgemäßen Verfahren einzusetzen. Ausgangsstoffe sind daher vorentschleimte, insbesondere wasserentschleimte Öle, die sich in der Regel durch einen Phosphorgehalt zwischen 50 und 300ppm auszeichnen. Nur ausnahmsweise besitzen vorentschleimte Öle höhere Phosphorgehalte, kaum jemals über 500ppm Phosphor.
Öle schwankender Qualität können auf der gleichen Anlage verarbeitet werden. Es ist auch möglich, teilentschleimte Öle, sowie Preßöle oder Extraktionsöle mit einzusetzen, und zwar in Abmischung mit vorentschleimten Ölen.. Ausnahmsweise kann der Phosphorgehalt dann ebenfalls über 500 ppm liegen.
Eine vorherige Trocknung des Öls ist entbehrlich.

Um das Enzym wirken lassen zu können, müssen beide Phasen, die Ölphase und die enzymhaltige Wasserphase innig miteinander vermischt werden. Bloßes Umrühren reicht bei weitem nicht aus.

Eine gute Verteilung des Enzyms im Öl ist gewährleistet, wenn es in einer geringen Wassermenge von 0.5-5 Gew.% (bezogen auf Öl) gelöst und in dieser Form in dem Öl zu Tröpfchen von weniger als 10 Mikrometer Durchmesser (Gewichtsmittelwert) emulgiert wird. Bevorzugt sind die Tröpfchen kleiner als 1 Mikrometer. Turbulentes Rühren mit Radialgeschwindigkeiten über 100 cm/sec hat sich bewährt. Stattdessen kann das Öl mit Hilfe einer externen Kreiselpumpe im Reaktor umgewälzt werden. Auch mittels Ultraschalleinwirkung läßt sich die enzymhaltige Wasserphase fein verteilen. Üblich ist ein Dispergierer wie z.B. Ultraturrax.

Die enzymatische Reaktion dürfte an der Grenzfläche zwischen der Ölphase und der Wasserphase stattfinden. Es ist das Ziel all dieser Maßnahmen zur Vermischung, für die enzymhaltige Wasserphase eine möglichst große Oberfläche zu erzeugen. Der Zusatz von Tensiden verstärkt die Feinverteilung der Wasserphase. Fallweise werden daher der Enzymlösung Tenside mit HLB-Werten von über 9, wie Na-Dodecylsulfat zugesetzt, wie in EP-A 0 513 709 beschrieben. Eine ähnlich wirksame Methode zur Verbesserung der Emulgierung ist der Zusatz von Lysolecithin. Die Zusatzmengen können bei 0.001% bis 1% bezogen auf Öl liegen. In allen Fällen werden beim erfindungsgemäßen Verfahren hochdisperse Wasser-in-ÖI-Emulsionen erzeugt.

Die Temperatur bei der Enzymbehandlung ist nicht kritisch. Temperaturen zwischen 20 und 80 C sind geeignet, letztere darf allerdings nur kurzzeitig angewandt werden. Die erfindungsgemäße Phospholipase zeichnet sich insgesamt durch eine gute Temperaturbeständigkeit aus. Sie wird durch den niedrigen Anwendungs-pH nicht beeinträchtigt. Optimal sind Anwendungstemperaturen von 30-50 C.

Die Behandlungsdauer hängt von der Temperatur ab und kann mit zunehmender Temperatur kürzer gehalten werden. Zeiten von 0,1 bis 10, vorzugsweise 1 bis 5 Stunden sind in der Regel ausreichend. Die Reaktion erfolgt in einem Entschleimungsreaktor, der in Etagen aufgeteilt werden kann, wie in DE-A 43 39 556 beschrieben.Somit ist neben einer batchweisen Fahrweise auch ein kontinuierlicher Betrieb möglich.
Die Reaktion kann dabei auch in verschiedenen Temperaturstufen durchgeführt werden. So kann z.B. 3 Stunden lang bei 40 C, danach 1 Stunde bei 60 C inkubiert werden. Ein stufenweiser Ablauf der Reaktion eröffnet auch die Möglichkeit, in den einzelnen Stufen verschiedenen pH-Werte einzustellen. So kann in einer ersten Stufe der pH-Wert der Lösung z.B. auf 7 eingestellt werden, in einer zweiten Stufe unter Zugabe von Citronensäure auf 2.5. In mindestens einer Stufe muß aber der pH-Wert der Enzymlösung erfindungsgemäß unter 3 liegen. Bei der nachträglichen pH-Einstellung unter diese erfindungsgemäße Marke wurde eine Wirkungsverschiechterung beobachtet. Daher wird die Citronensäure bevorzugt der Enzymlösung vor deren Einmischung ins Öl zugesetzt.

Nach Abschluß der Enzymbehandlung wird die Enzymlösung mitsamt der darin aufgenommenen Abbauprodukte der NHP - batchweise oder kontinuierlich - von der Ölphase abgetrennt, vorzugsweise durch Zentrifugieren. Da sich die Enzyme durch eine hohe Stabilität auszeichnen und die Menge der aufgenommenen Abbauprodukte gering ist - sie fallen als Schlamm aus - kann die gleiche wäßrige Enzymphase mehrmals wiederverwendet werden. Es besteht auch die Möglichkeit, entsprechend DE-A 43 39 556 das Enzym vom Schlamm abzulösen, sodaß eine weitgehend schlammfreie Enzymlösung wiedereingesetzt werden kann.

Mit dem erfindungsgemäßen Verfahren der Entschleimung werden Öle erhalten, die weniger als 15 ppm Phosphor enthalten. Das eigentliche Ziel sind Phosphorgehalte von weniger als 10 ppm; im Idealfall sollten es weniger als 5ppm sein. Mit Phosphorgehalten unter 10ppm ist die Weiterverarbeitung des Öls nach dem Verfahren der destillativen Entsäuerung ohne weiteres möglich.
Im Zuge des erfindungsgemäßen Verfahrens werden auch eine Reihe andere lonen wie Magnesium, Calcium, Zink, sowie auch Eisen weitgehend aus dem Öl entfernt. Dank des erreichten niedrigen Eisengehaltes, meist unter 0.1 ppm, hat das Produkt ideale Voraussetzungen für eine gute Oxidationbeständigkeit bei der Weiterverarbeitung und bei der Lagerung.

### Beispiel 1

500g naßentschleimtes Sojaöl mit einem Rest-Phosphorgehalt von 190ppm wird in einem Rundkolben auf 40 C erwärmt. Dazu gibt man 26 g Wasser, in dem 5 g Citronensäure und 0.19 g eines pulverförmigen Enzympräparats gelöst worden waren. Das Enzympräparat stammt aus einer Produktionscharge einer Aspergillus niger-Fermentation und enthält 60 Phospholipase Einheiten (=Lecitase-Units, LU) pro g. 1 Lecitase-Unit (LU) ist jene Enzymmenge, die bei 40 C, pH=8, aus Eigelb in einer Minute 1 Mikromol Fettsäure freisetzt. Das Enzympräparat wurde auch auf Lysophospholipase-Aktivität überprüft. Es wurden 1001 Lysophospholipase-Einheiten (=Lysolecitase-Units, LLU). pro g gemessen. 1 Lysolecitase-Unit ist jene Enzymmenge, die bei 55 C, pH=4.5, aus einer Lysolecithin-Emulsion 1 Mikromol Fettsäure pro Minute freisetzt. Das Enzympräparat, das im Hinblick auf den Gehalt an Phospholipasen nicht extra aufgereinigt wurde, enthält also neben Phospholipase A₂ eine bemerkenswert hohe Aktivität an Lysophospholipase und könnte als Phospholipase B.bezeichnet werden.

Der Inhalt des Rundkolbens wird mittels einer externen Kreiselpumpe intensiv dispergiert. Dabei wird der Inhalt des Kolbens etwa einmal pro Minute durchgesetzt. Die Wasserphase liegt dabei in einer Teilchengröße von unter 1µm vor. In Zeitabständen von einer Stunde werden Proben genommen und auf Phosphorgehalt untersucht. Dabei ergaben sich folgende Werte:

| Zeit in Stunden | 0 | 2 | 4 | 6 | 20 |
|---|---|---|---|---|---|
| | | | | | |
| Phosphorgehalt in ppm | 190 | 81 | 27.9 | 5.4 | 4.2 |

Der für eine nachfolgende destillative Entsäuerung benötigte niedrige Phosphorgehalt von <10ppm wurde durch das erfindungsgemäße Verfahren in 6 Stunden erreicht.

### Vergleichsversuch 1

Es wird wie in Beispiel 1 gearbeitet, nur wird anstelle des Enzympräparats eine entsprechende Menge an Molkenprotein, also nichtenzymatisches Protein zugesetzt. Die nach den gleichen Behandlungszeiten wie oben genommenen Proben zeigen, daß die Phosphorgehalte durch eine enzymfreie Behandlung nicht unter 121 ppm absinken. Citronensäure-Zusatz allein genügt demnach nicht. Das erhaltenen Öl ist für eine destillative Entsäuerung nicht geeignet.

| Zeit in Stunden | 0 | 2 | 4 | 6 | 20 |
|---|---|---|---|---|---|
| | | | | | |
| Phosphorgehalt in ppm | 190 | 152 | 128 | 123 | 121 |

### Vergleichsversuch 2

Es wird wie in Beispiel 1 gearbeitet, nur wird anstelle der Phospholipase aus Aspergillus eine reine Lysophospholipase des Handels (G-Zyme, von Enzyme Biosystems, USA, 1172 LLU pro g) eingesetzt. Sie besitzt keine Phospholipase A₂-Aktivität. Die nach den gleichen Behandlungszeiten wie oben genommenen Proben zeigen, daß die Phosphorgehalte durch den Einsatz von Lysophospholipase allein unter den vorgegebenen Bedingungen nicht unter 85ppm absinken. Das erhaltenen Öl ist für eine destillative Entsäuerung nicht geeignet.

| Zeit in Stunden | 0 | 2 | 4 | 6 | 20 |
|---|---|---|---|---|---|
| | | | | | |
| Phosphorgehalt in ppm | 190 | 138 | 124 | 119 | 85 |

## Patentansprüche

1. Verfahren zur Verminderung von in pflanzlichen Ölen enthaltenen phosphorhaltigen Bestandteilen durch deren enzymatischen Abbau mit acylspaltender Phospholipase,
**dadurch gekennzeichnet,**
**dass** ein aus Aspergillus stammendes Enzym eingesetzt wird, wobei das Enzym sowohl Phospholipase A₂-Aktivität (EC - Nr. 3.1.1.4) und/oder Phospholipase A₁-Aktivität (EC - Nr. 3.1.1.32), als auch Lysophospholipase-Aktivität (EC - Nr. 3.1.1.5) enthält und der pH-Wert der eingesetzten Enzymlösung auf < 3 eingestellt wird.

2. Verfahren nach Anspruch 1, **dadurch gekennzeichnet, dass** der pH-Wert mit Citronensäure eingestellt wird.

3. Verfahren nach einem der Ansprüche 1 oder 2, **dadurch gekennzeichnet, dass** es bei Temperaturen von 20 bis 80°C, bevorzugt bei 30 bis 50°C, durchgeführt wird.

4. Verfahren nach einem oder mehreren der Ansprüche 1 bis 3, **dadurch gekennzeichnet, dass** die das Enzym enthaltende Wasserphase im Öl zu Tröpfchen einer Größe unter 10 Mikrometer emulgiert wird.

5. Verfahren nach einem oder mehreren der Ansprüche 1 bis 4, **dadurch gekennzeichnet, dass** zumindest teilweise vorentschleimtes, insbesondere nassentschleimtes Öl eingesetzt wird.

6. Verfahren nach Anspruch 5, **dadurch gekennzeichnet, dass** ein Phosphorgehalt von 50 bis 500 ppm im Öl auf weniger als 15 ppm, bevorzugt auf weniger als 10 ppm, bevorzugter auf weniger als 5 ppm reduziert wird.

7. Verfahren nach Anspruch 6, **dadurch gekennzeichnet, dass** das pflanzliche Öl Sojaöl ist.

8. Verfahren nach Anspruch 6, **dadurch gekennzeichnet, dass** das pflanzliche Öl Raps- oder Sonnenblumenöl ist.

9. Verfahren nach einem oder mehreren der Ansprüche 1 bis 8, **dadurch gekennzeichnet, dass** die wässrige Lösung der Phospholipase nach der Einwirkung von dem behandelten Öl abgetrennt und erneut verwendet wird.

10. Verfahren nach einem oder mehreren der Ansprüche 1 bis 9, **dadurch gekennzeichnet, dass** es chargenweise durchgeführt wird.

11. Verfahren nach einem oder mehreren der Ansprüche 1 bis 9, **dadurch gekennzeichnet, dass** es kontinuierlich durchgeführt wird.

12. Verfahren nach einem oder mehreren der Ansprüche 1 bis 11, **dadurch gekennzeichnet, dass** mit der Verminderung des Gehalts an phosphorhaltigen Bestandteilen auch der Eisengehalt des Öls vermindert wird.

## Claims

1. Method of reducing phosphorus-containing ingredients present in vegetable oils by breaking them down enzymatically with acyl-cleaving phospholipase, **characterised in that** an enzyme obtained from Aspergillus is used, the enzyme containing both phospholipase A₂ activity (EC - No. 3.1.1.4) and/or phospholipase A₁-activity (EC - No. 3.1.1.32) as well as lysophospholipase activity (EC - No. 3.1.1.5) and the pH of the enzyme solution used being adjusted to < 3.

2. Method according to claim 1, **characterised in that** the pH is adjusted with citric acid.

3. Method according to one of claims 1 or 2, **characterised in that** it is carried out at temperatures of 20 to 80°C, preferably 30 to 50°C.

4. Method according to one or more of claims 1 to 3, **characterised in that** the aqueous phase containing the enzyme is emulsified in oil to form droplets less than 10 microns in size.

5. Method according to one or more of claims 1 to 4, **characterised in that** oil is used which has been at least partially deslimed, particularly wet deslimed, beforehand.

6. Method according to claim 5, **characterised in that** a phosphorus content of from 50 to 500 ppm in oil is reduced to less than 15 ppm, preferably to less than 10 ppm, more preferably to less than 5 ppm.

7. Method according to claim 6, **characterised in that** the vegetable oil is soya oil.

8. Method according to claim 6, **characterised in that** the vegetable oil is rapeseed or sunflower oil.

9. Method according to one or more of claims 1 to 8, **characterised in that** after the treatment the aqueous solution of the phospholipase is separated from the treated oil and used again.

10. Method according to one or more of claims 1 to 9, **characterised in that** it is carried out batchwise.

11. Method according to one or more of claims 1 to 9, **characterised in that** it is carried out continuously.

12. Method according to one or more of claims 1 to 11, **characterised in that** the iron content of the oil is also reduced as the content of phosphorus-containing ingredients is reduced.

## Revendications

1. Procédé pour diminuer les constituants phosphorés contenus dans les huiles végétales, par leur dégradation enzymatique avec une phospholipase dissociant les groupes acyle, **caractérisé en ce qu'**on utilise une enzyme provenant d'Aspergillus, l'enzyme contenant tant une activité de phospholipase A₂ (EC - N° 3.1.1.4) et une activité de phospholipase A₁ (EC - N° 3.1.1.32) qu'une activité de lysophospholipase (EC - N° 3.1.1.5), le pH de la solution enzymatique utilisée étant ajusté à une valeur < 3.

2. Procédé selon la revendication 1, **caractérisé en ce que** le pH est ajusté avec de l'acid citrique.

3. Procédé selon l'une des revendications 1 ou 2, **caractérisé en ce qu'**il est mis en oeuvre à des températures de 20 à 80°C, de préférence de 30 à 50°C.

4. Procédé selon Tune ou plusieurs des revendications 1 à 3, **caractérisé en ce que** la phase aqueuse contenant l'enzyme est émulsionnée dans l'huile pour donner des gouttelettes ayant une grosseur inférieure à 10 micromètres.

5. Procédé selon Tune ou plusieurs des revendications 1 à 4, **caractérisé en ce qu'**on utilise une huile au moins partiellement pré-démucilaginée, en particulier démucilaginée par voie humide.

6. Procédé selon la revendication 5, **caractérisé en ce qu'**une teneur de l'huile en phosphore de 50 à 500 ppm est réduite à une valeur inférieure à 15 ppm, de préférence inférieure à 10 ppm, plus particulièrement inférieure à 5 ppm.

7. Procédé selon la revendication 8, **caractérisé en ce que** l'huile végétale est l'huile de soja.

8. Procédé selon la revendication 6, **caractérisé en ce que** l'huile végétale est l'huile de colza ou l'huile de tournesol.

9. Procédé selon l'une ou plusieurs des revendications 1 à 8, **caractérisé en ce que** la solution aqueuse de la phospholipase est séparée de l'huile traitée après son action, et est utilisée de nouveau.

10. Procédé selon l'une ou plusieurs des revendications 1 à 9, **caractérisé en ce qu'**il est mis en oeuvre dune manière discontinue.

11. Procédé selon Tune ou plusieurs des revendications 1 à 9, **caractérisé en ce qu'**il est mis en oeuvre dune manière continue.

12. Procédé selon Tune ou plusieurs des revendications 1 à 11, **caractérisé en ce que** la diminution de la teneur en constituants phosphorés s'accompagne aussi d'une diminution de la teneur en fer de l'huile.
